(19) Europäisches Patentamt
European Patent Office
Office européen des brevets

(11) EP 2 650 873 B1

(12) EUROPEAN PATENT SPECIFICATION

(45) Date of publication and mention of the grant of the patent:
**21.06.2017 Bulletin 2017/25**

(21) Application number: **10860523.9**

(22) Date of filing: **10.12.2010**

(51) Int Cl.:
***G10L 25/51*** *(2013.01)* ***A61F 5/56*** *(2006.01)*
***A61B 7/00*** *(2006.01)*

(86) International application number:
**PCT/JP2010/072290**

(87) International publication number:
**WO 2012/077239 (14.06.2012 Gazette 2012/24)**

# (54) ACOUSTIC SIGNAL PROCESSING DEVICE, ACOUSTIC SIGNAL PROCESSING METHOD, AND ACOUSTIC SIGNAL PROCESSING PROGRAM

VORRICHTUNG ZUR VERARBEITUNG AKUSTISCHER SIGNALE, AKUSTISCHES SIGNALVERARBEITUNGSVERFAHREN UND AKUSTISCHES SIGNALVERARBEITUNGSPROGRAMM

DISPOSITIF DE TRAITEMENT DE SIGNAL ACOUSTIQUE, PROCÉDÉ DE TRAITEMENT DE SIGNAL ACOUSTIQUE, ET PROGRAMME DE TRAITEMENT DE SIGNAL ACOUSTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**16.10.2013 Bulletin 2013/42**

(73) Proprietor: **Fujitsu Limited**
**Kawasaki-shi, Kanagawa 211-8588 (JP)**

(72) Inventors:
- **TANAKA, Masakiyo**
  **Kawasaki-shi, Kanagawa 211-8588 (JP)**
- **OTANI, Takeshi**
  **Kawasaki-shi, Kanagawa 211-8588 (JP)**
- **SUZUKI, Masanao**
  **Kawasaki-shi, Kanagawa 211-8588 (JP)**

(74) Representative: **Hoffmann Eitle**
**Patent- und Rechtsanwälte PartmbB**
**Arabellastraße 30**
**81925 München (DE)**

(56) References cited:
**WO-A1-2004/104960 CA-A1- 1 220 283**
**JP-A- 7 028 486 JP-A- 2007 511 812**
**JP-U- H0 396 078 US-A1- 2010 283 618**

- **CAVUSOGLU M ET AL: "An efficient method for snore/nonsnore classification of sleep sounds", PHYSIOLOGICAL MEASUREMENT, INSTITUTE OF PHYSICS PUBLISHING, BRISTOL, GB, vol. 28, no. 8, 1 August 2007 (2007-08-01) , pages 841-853, XP020120816, ISSN: 0967-3334, DOI: 10.1088/0967-3334/28/8/007**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 650 873 B1

## Description

[Technical Field]

The embodiments discussed herein are directed to an acoustic signal processing apparatus, an acoustic signal processing method, and an acoustic signal processing program.

[Background Art]

Conventionally, there is a known technology for improving user's sleep by detecting an acoustic signal rear a sleeping user. For example, there is an acoustic signal processing apparatus that detects user's snoring and causes the user to stop snoring. For example, when the volume of an acoustic signal detected near a user is equal to or greater than a threshold, the acoustic signal processing apparatus detects the corresponding acoustic signal as snoring. Then, when the number of detections of snoring reaches a predetermined number, the acoustic signal processing apparatus wakes the user up by sound or vibration to cause the user to stop snoring.

With reference to FIG. 18, a case will be explained below in which a conventional acoustic signal processing apparatus detects snoring. FIG. 18 is a diagram for explaining the conventional technology. In FIG. 18, the vertical axis represents the volume of an acoustic signal and the horizontal axis represents time. Values on the horizontal axis represent the number of detections of the acoustic signal. In the example illustrated in FIG. 18, it is assumed that a threshold for the volume is denoted by Pw and the threshold for the number of detections is three. As illustrated in FIG. 18, for example, the acoustic signal processing apparatus compares the volume of an acoustic signal with the threshold Pw at every detection of the acoustic signal, and detects the first, the third, and the sixth acoustic signals as snoring. When the number of 5 detections of snoring reaches three, that is, when the sixth acoustic signal is detected, the acoustic signal processing apparatus wakes the user up by using sound or vibration.

[Citation List]

[Patent Citation]

Patent Document 1: Japanese Examined Utility Model Application Publication No. 3036078

CA 1 220 283 A1 relates to a speech signal detector which has threshold values adapted to the fluctuating level of noise contained in input speech signals. It comprises an absolute value detector for detecting the absolute value of each of the input signals; a noise power detector for calculating from the output of the absolute value detector the noise power contained in each input signal; a first threshold value setting circuit for generating a first threshold value from the output of the noise power detector circuit; a level detector for comparing the output of the absolute value detector and the threshold value supplied by the first threshold value setting circuit; an accumulating circuit for accumulating the outputs of the level detector; a comparator for comparing the output value of the accumulating circuit and a second threshold value.

US 2010/283618 A1 relates to a health-sensing device including a sensor, a filter, and a transmitter. The sensor may be configured to sense one or more factors relating to an indicator of a health related condition or occurrence such as snoring and may include one or more microphone devices, accelerometers, and/or MEMs devices. The filter may be configured to evaluate a signal from the sensor and determine if the indicator has been detected. The transmitter may be arranged for initiating a transmission based on a signal from the filter. The health-action device may be configured for responding to an indicator of a health related condition or occurrence of a user and may include one or more of a receiver, a processor, and a responder.

[Summary of Invention]

[Technical Problem]

However, the conventional technology as described above has a problem in that is difficult to accurately determine annoying snoring.

For example, in the conventional technology, a "detection failure" occurs, in which even when other people subjectively perceives a detected acoustic signal as snoring, the acoustic signal with the volume lower than the threshold is not detected as snoring. In the example illustrated in FIG. 18, even when other people subjectively perceive the second acoustic signal as snoring, the 25 conventional acoustic signal processing apparatus does not detect the second acoustic signal as snoring. Even if the threshold for the volume is lowered, the same problem occurs with respect to the lowered threshold.

Furthermore, the volume of an acoustic signal 30 generated by the user is not always constant, but varies such that the volume becomes higher or lower. Therefore, in the conventional technology, a "detection delay" occurs, in which even when other people subjectively perceive user's snoring as annoying snoring, the user's snoring is not detected as snoring until the number of detections of snoring reaches a predetermined number and detection is delayed. In the example illustrated in FIG. 18, even if other people subjectively perceive user's snoring as annoying when the third acoustic signal is detected, the conventional acoustic signal processing apparatus does not detect the user's snoring until the sixth acoustic signal is detected. Sven if the threshold for the n-amber of detections is lowered, the same problem occurs with respect to the lowered threshold. The above problem occurs not only when snoring is detected, but also when, in the same manner, an acoustic signal, such as teeth grinding or monology, generated by a human body is detected.

The disclosed technology has been made in view of the above circumstances, and an object is to provide an acoustic signal processing apparatus, an acoustic signal processing method, and an acoustic signal processing program capable of accurately determining an acoustic signal.

[Solution to Problem]

This object is accomplished by the subject-matter of the independent claims. The dependent claims describe advantageous embodiments.

In order to solve the above problems and to achieve the object, in the disclosed apparatus, a detecting unit detects an acoustic signal that is contained in a sound input signal and that is generated by a human body. Then, a calculating unit calculates an effective volume indicating an effective volume of the acoustic signal detected by the detecting unit, based on the input signal. Then, an accumulating unit accumulates the effective volumes calculated by the calculating unit to calculate an accumulated effective volume. Then, a determining unit determines whether the accumulated effective volume calculated by the accumulating unit has reached a predetermined threshold.

[Advantageous Effects of Invention]

According to one embodiment of the technology disclosed herein, it is possible to accurately determine an acoustic signal.

[Brief Description of Drawings]

FIG. 1 is a block diagram illustrating an example of a configuration of an acoustic signal processing apparatus according to a first embodiment.
FIG. 2 is a diagram for explaining a degree of annoyance.
FIG. 3 is a diagram for explaining the degree of annoyance.
FIG. 4 is a diagram for explaining a relationship between the volume of snoring and a subjective evaluation value.
FIG. 5 is a diagram for explaining a relationship between the number of times of snoring and the subjective evaluation value.
FIG. 6 is a block diagram illustrating an example of a configuration of a detecting unit according to the first embodiment.
FIG. 7 is a diagram for explaining characteristics of snoring.
FIG. 8 is a diagram for explaining characteristics of snoring.
FIG. 9 is a diagram for explaining a process performed by the duration calculating unit.
FIG. 10 is a diagram illustrating an example of a detection result obtained by the detecting unit.
FIG. 11 is a diagram for explaining a method for calculating the degree of annoyance.
FIG. 12 is a diagram illustrating an example of a calculation result obtained by a calculating unit.
FIG. 13 is a diagram illustrating an example of an accumulation result obtained by an accumulating unit.
FIG. 14 is a flowchart of the flow of a process performed by the acoustic signal processing apparatus according to the first embodiment.
FIG. 15 is a block diagram illustrating an example of a configuration of a mobile terminal according to a second embodiment.
FIG. 16 is a diagram for explaining a method for calculating the degree of annoyance.
FIG. 17 is a diagram illustrating an example of a computer that executes an acoustic signal processing program.
FIG. 18 is a diagram for explaining a conventional technology.

[Embodiment for Carrying Out the Invention]

Preferred embodiments of an acoustic signal processing apparatus, an acoustic signal processing method, and an acoustic signal processing program of the present application will be explained in detail below with reference to accompanying drawings. The present application is not limited to the embodiments. The embodiments can be arbitrarily combined within a scope that does not contradict the processing contents.

First Embodiment

An example of a configuration of an acoustic signal processing apparatus according to a first embodiment will be explained. FIG. 1 is a block diagram illustrating an example of the configuration of the acoustic signal processing apparatus according to the first embodiment. As illustrated in FIG. 1, an acoustic signal processing apparatus 100 includes an input memory 110, a detecting unit 120, a calculating unit 130, an accumulation memory 140, an accumulating unit 150, and a determining unit 160. The acoustic signal processing apparatus 100 accumulates degrees of annoyance that are calculated based on the volume of snoring, and determines annoying snoring. In the following, the degree of annoyance is explained.

FIG. 2 and FIG. 3 are diagrams for explaining the degree of annoyance. In FIG. 2 and FIG. 3, the vertical axis represents an accumulated degree of annoyance and the horizontal axis represents time. The value on the horizontal axis represents the number of detections of snoring. In the example illustrated in FIG. 2 and FIG. 3, a threshold is denoted by Pw. FIG. 2 illustrates an example in which the degree of annoyance calculated per snoring is large because the volume of snoring is high, and FIG. 3 illustrates an example ion which the degree of annoyance calculated per snoring is small because the volume of snoring is low. As illustrated in FIG. 2, when the volume of snoring is high, the number of times of snoring until the accumulated degree of annoyance reaches the threshold Pw is, for example, three. In this case, the acoustic signal processing apparatus 100 determines that the user’s snoring is annoying upon detection of the third snoring. Incidentally, as illustrated in FIG. 3, when the volume of snoring is low, the number of times of snoring until the accumulated degree of annoyance reaches the threshold Pw is, for example, seven. In this case, the acoustic signal processing apparatus 100 determines that the user’s snoring is annoying upon detection of the seventh snoring.

Meanwhile, the degree of annoyance is used for the determination because the inventors have learned that the volume of snoring and the number of times of snoring have influences on whether a person other than a user perceive the snoring as annoying. FIG. 4 is a diagram for explaining a relationship between the volume of snoring and a subjective evaluation value. The subjective evaluation value is a score representing subjective evaluation made by other people when the other people hear the snoring. In FIG. 4, evaluation of "not annoyed" is represented by "2", evaluation of "rather not annoyed" is represented by "1", evaluation of "no opinion" is represented by "0", evaluation of "annoyed" is represented by "1", and evaluation of "very annoyed is represented by "2". In FIG. 4, the vertical axis represents the subjective evaluation value and the horizontal axis represents the volume of snoring. FIG. 4 illustrates a plot of experimental data representing the average of subjective evaluation values provided by the test subjects when the number of times of snoring is fixed to five and the volume of snoring is changed. As illustrated in FIG. 4, there is a relationship between the volume of snoring and the subjective evaluation value such that the subjective evaluation value decreases as the volume of snoring increases. Namely, the experimental data illustrated in FIG. 4 indicates that the increased volume of snoring increases the annoyance of the other people.

FIG. 5 is a diagram for explaining a relationship between the number of times of snoring and the subjective evaluation value. In FIG. 5, the vertical axis represents the subjective evaluation value and the horizontal axis represents the number of times of snoring. FIG. 5 illustrates a plot of experimental data representing the average of the subjective evaluation values provided by the test subjects when the volume of snoring is fixed to 70 dBA and the number of times of snoring is changed. As illustrated in FIG. 5, there is a relationship between the number of times of snoring and the subjective evaluation value such that the subjective evaluation value decreases as the number of times of snoring increases. Namely, the experimental data illustrated in FIG. 5 indicates that the increased number of times of snoring increases the annoyance of the other people. The acoustic signal processing apparatus 100 illustrated in FIG. 1 accumulates the degree of annoyance calculated based on the volume of snoring, to thereby determine annoying snoring.

Referring back to the explanation of FIG. 1, the input memory 110 accumulates a sound input signal. For example, the input memory 110 accumulates, as input signal data, input signal data divided into a plurality of frames with a predetermined sample size, in association with a frame identification number. The sound input signal accumulated by the input memory 110 is, for example, an input signal of sound collected by a sound collecting device, such as a microphone, near a sleeping user.

The detecting unit 120 detects an acoustic signal that is generated by a human body and that is contained in the sound input signal. For example, when an input signal similar to a past input signal is continued for a predetermined time, the detecting unit 120 detects a corresponding input signal as snoring. FIG. 6 is a block diagram illustrating an example of a configuration of the detecting unit according to the first embodiment. As illustrated in FIG. 6, the detecting unit 120 includes an FFT (Fast Fourier Transform) unit 121, a power spectrum calculating unit 122, a similarity calculating unit 123, a duration calculating unit 124, and a snoring determining unit 125. The snoring is one example of the acoustic signal.

The detecting unit 120 uses the characteristics of snoring in order to detect snoring. FIG. 7 and FIG. 8 are diagrams for explaining the characteristics of snoring. In FIG. 7, the vertical axis represents the magnitude of the frequency of an input signal and the horizontal axis represents time. In the example illustrated in FIG. 7, signals present in respective time periods 7a, 7b, 7c, 7d, and 7e correspond to snoring. As illustrated in FIG. 7, snoring is repeated at a cycle T1. For example, T1 is about 3 to 5 seconds. Furthermore, each snoring continues for a time T2. For example, T2 is about 0.4 to 2 seconds. In FIG. 8, the vertical axis represents the power of the input signal and the horizontal axis represents the frequency. In the example illustrated in FIG. 8, a waveform 8a indicated by a solid line corresponds to the frequency characteristic of one frame in the time period 7a, and a waveform 8b indicated by a dashed line corresponds to the frequency characteristic of one frame in the time period 7b. As illustrated in FIG. 8, the waveform 8a and the waveform 8b are similar to each other. Namely, the frequency characteristics at respective times of snoring are similar to one another. In the following, a process performed by the detecting unit 120 to detect snoring by using the characteristics of the snoring will be explained.

Referring back to the explanation of FIG. 6. The FFT unit 121 transforms the sound input signal into a signal in the frequency domain by Fourier transform. For example, the FFT unit 121 acquires an input signal that is divided into a plurality of frames with a predetermined sample size, and performs Fourier transform on the input signal of the acquired frame, to thereby generate a signal in the frequency domain for each of the frames. The FFT unit 121 outputs the signal in the frequency domain generated for each frame to the power spectrum calculating unit 122. The sound input signal input to the FFT unit 121 is, for example, an input signal of sound collected by a sound collecting device, such as a microphone, near a sleeping user.

The power spectrum calculating unit 122 calculates the power spectrum from the signal in the frequency domain transformed by the FFT unit 121. For example, the power spectrum calculating unit 122 calculates the sum of squares of the real part and the imaginary part of each band for the signal in the frequency domain of the current frame received by the FFT unit 121, to thereby calculate the power spectrum of each band. Then, the power spectrum calculating unit 122 outputs the calculated power spectrum of each band to the similarity calculating unit 123.

The similarity calculating unit 123 calculates the similarity between a current power spectrum and a past power spectrum. The similarity is a value indicating a resemblance between the current power spectrum and the past power spectrum. For example, the value increases as the resemblance between the waveform 8a and the waveform 8b illustrated in FIG. 8 increases. For example, when receiving the power spectrum at a current time "t" from the power spectrum calculating unit 122, the similarity calculating unit 123 selects, as the past power spectrum, a power spectrum at a time "t-x" that is backward by a time "x" from the time "t". The similarity calculating unit 123 calculates a difference between the selected past power spectrum and the current power spectrum for each band, and determines whether the calculated difference is equal to or smaller than a threshold. When determining that the calculated difference is equal to or smaller than the threshold, the similarity calculating unit 123 adds "1" to the similarity. On the other hand, when determining that the calculated difference is greater than the threshold, the similarity calculating unit 123 adds "0" to the similarity. The similarity calculating unit 123 performs the above process on all of the bands to calculate the degree of similarity for each of the bands. Then, the similarity calculating unit 123 adds up the calculated degrees of similarity of all the bands, to thereby calculate the degree of similarity between the power spectrum at the time "t" and the power spectrum at the time "t-x". The threshold is, for example, 3dB. The threshold is not limited to this example, but may be set to an arbitrary value by a user using the acoustic signal processing apparatus 100.

Furthermore, the similarity calculating unit 123 calculates the degree of similarity by changing the time "x" from "x1" to "x2". Namely, the similarity calculating unit 123 calculates the similarity between the power spectrum at each time ranging from a time "t-x1" to a time "t-x2" and the power spectrum at the time "t". For example, the time "x" corresponds to the cycle T1 illustrated in FIG. 7, x1 = 3 seconds, and x2 = 5 seconds. The similarity calculating unit 123 outputs the calculated similarity to the duration calculating unit 124 as the similarity of the frame at the time "t". The similarity calculating unit 123 stores therein the past power spectrum.

The duration calculating unit 124 calculates a duration of the similarity. FIG. 9 is a diagram for explaining a process performed by the duration calculating unit. FIG. 9 is a table containing the similarities, where the horizontal axis represents the current time "t" and the vertical axis represents the time "x" backward from the current time. A shaded area in FIG. 9 indicates that an identifier F is stored. The identifier F indicates that the similarity of the frame at the time "t" is equal to or greater than the threshold. As illustrated in FIG. 9, for example, when receiving the similarity of the frame at the current time "t" from the similarity calculating unit 123, the duration calculating unit 124 stores the received similarity in the table. For example, when the similarity between the power spectrum at the current time "t" and the power spectrum at the tine "t-x1" is "2", the duration calculating unit 124 stores "2" in a lower left field of the table. Similarly, the duration calculating unit 124 stores the similarities at other times "x". The duration calculating unit 124 determines whether the stored similarity is equal to or greater than the threshold. When determining that the degree of similarity is equal to or greater than the threshold, the duration calculating unit 124 stores the identifier F in a corresponding field of the table. The threshold is, for example, "10". The threshold is not limited to this example, but may be set to an arbitrary value by a user using the acoustic signal processing apparatus 100.

Furthermore, when receiving the similarity of the frame at a next time "t+1" from the similarity calculating unit 123, the duration calculating unit 124 stores the received similarity in the table and determines whether the stored similarity is equal to or greater than the threshold in the same manner as described above. When the identifier F continues from the time "t" to the time "t+1" with respect to the same time "x", the duration calculating unit 124 repeats the above process. The duration calculating unit 124 repeats the above process until the continuation of the identifier F is slopped. In the example illustrated in FIG. 9, a process on the frame at a time "t+7" is performed and the above process is repeated until the continuation of the identifier F is stopped.

When the continuation of the identifier F is stopped, the duration calculating unit 124 counts the number of the continued identifiers F. The duration calculating unit 124 converts the counted number of the continued identifiers F into a duration, and outputs the duration to the snoring determining unit 125. In the example illustrated in FIG. 9, the identifier F is continued seven times in seven frames from the time "t" to the time "t+6". The duration calculating unit 124 outputs, as a duration, a time corresponding to the consecutive frames to the snoring determining unit 125. If the identifier F is continued in a plurality of times "x", the duration calculating unit 124 performs the above process with respect to the greatest number of the continued identifiers F.

Referring back to the explanation of FIG. 6, the snoring determining unit 125 determines presence or absence of snoring. For example, the snoring determining unit 125 determines whether the duration received from the duration calculating unit 124 is within a tine range "y". The time range "y" corresponds to, for example, the time T2 illustrated in FIG. 7, and is about C.4 to 2 seconds. When determining that the duration is within the time range "y", the snoring determining unit 125 determines that the sound input signal corresponding to a subject duration contains snoring. Then, the snoring determining unit 125 determines, as a snoring interval, an interval corresponding to the input signal that is determined to contain snoring.

In this way, the detecting unit 120 detects snoring from the sound input signal by using the characteristics of snoring. FIG. 10 is a diagram illustrating an example of a detection result obtained by the detecting unit. In FIG. 10, the vertical axis represents volume and the horizontal axis represents time. Values on the horizontal axis represent the number of detections of snoring. As illustrated in FIG. 10, for example, the detecting unit 120 detects a snoring interval corresponding to one snoring. The snoring interval is information containing information on a frame in which snoring starts and information on a frame in which the snoring stops. The detecting unit 120 outputs the detected snoring interval to the calculating unit 130 every time the detecting unit 120 detects the snoring interval.

Referring back to the explanation of FIG. 1, the calculating unit 130 calculates a degree of annoyance with the snoring detected by the detecting unit 120, based on the sound input signal. For example, the calculating unit 130 acquires input signal data corresponding to the snoring, interval detected by the detecting unit 120 from the input memory 110. The calculating unit 130 calculates a volume of snoring by using the acquired input signal data. As the volume of snoring, for example, an average volume is used that is calculated as a mean square value of a power value of the snoring interval. For example, the calculating unit 130 calculates the volume of snoring by using Equation (1) below.

$$fpow(n) = \frac{\sum_{k=0}^{N-1} sample(k)^2}{N} \qquad (1)$$

In Equation (1), n represents a frame number. fpow(n) represents a volume of an n-th frame. sample(k) represents a k-th sample. N represents a frame length. For example, the calculating unit 130 calculates an average volume of each of the frames contained in the snoring interval by using Equation (1), and adds up the calculated average volumes of the respective frames, to thereby calculate the volume of snoring.

Furthermore, the calculating unit 130 calculates the degree of annoyance with snoring based on the volume of snoring. For example, the calculating unit 130 calculates the degree of annoyance with snoring by using Equation (2) below.

$$\mathrm{Annoy}(n) = \begin{cases} A\min & (\mathrm{fpow}(n) < TH1) \\ AMax \times \dfrac{\mathrm{fpow}(n) - TH1}{TH2 - TH1} & (TH1 \le \mathrm{fpow}(n) \le TH2) \\ AMax & (\mathrm{fpow}(n) > TH2) \end{cases} \qquad (2)$$

In Equation (2), n represents the number of times of snoring. Annoy(n) represents the degree of annoyance with n-th snoring. fpow(n) represents the volume of n-th snoring. AMax represents a predetermined maximum value of the degree of annoyance, and is set to "100" for example. Amin represents a predetermined minimum value of the degree of annoyance, and is set to "0" for example. TH1 and TH2 represent thresholds. For example, TH1 is set to "60dBA" and TH2 is set to "80dBA". For example, AMax corresponds to a minimum value of the volume of single snoring that causes other person to feel annoyed, and is set to the same value as the threshold set by the determining unit 160 as will be described later. However, the method to set AMax is not limited to the setting method described herein. For example, to prevent the determining unit 160 from erroneously determining that the degree of annoyance reaches the threshold when loud sound other than snoring is detected, AMax may be set to a value smaller than the threshold set by the determining unit 160. Furthermore, AMax, Amin, TH1, and TH2 are also not limited to the example described herein, but may be set to arbitrary values by a user using the acoustic signal processing apparatus 100.

The method for calculating the degree of annoyance using Equation (2) by the calculating unit 130 will be explained below. FIG. 11 is a diagram for explaining the method for calculating the degree of annoyance. In FIG. 11, the vertical axis represents the degree of annoyance and the horizontal axis represents a volume (dBA). As illustrated in FIG. 11, for example, when the volume fpow (n) is equal to or greater than the threshold TH1 and smaller than the threshold TH2, the calculating unit 130 calculates, as the degree of annoyance Annoy(n), a value proportional to the volume fpow(n). Furthermore, when the volume fpow(n) is smaller than the threshold TH1, the calculating unit 130 calculates Amin as the degree of annoyance Annoy (n). Moreover, when the volume fpow(n) is equal to or greater than the threshold TH2, the calculating unit 130 calculates Amax as the degree of annoyance Annoy (n).

FIG. 12 is a diagram illustrating an example of a calculation result obtained by the calculating unit. In FIG. 12, the vertical axis represents the degree of annoyance and the horizontal axis represents time. Values on the horizontal axis represent the number of detections of snoring. As illustrated in FIG. 12, for example, the calculating unit 130 calculates the degree of annoyance with n-th snoring every time the calculating unit 130 detects n-th snoring from the detecting unit 120. The calculating unit 130 outputs the calculated degree of annoyance to the accumulating unit 150 every time the calculating unit 130 calculates the degree of annoyance with the n-th snoring. The degree of annoyance is an example of an effective volume.

Referring back to the explanation of FIG. 1, the accumulation memory 140 accumulates an accumulated degree of annoyance that has been accumulated by the accumulating unit 150 to be described later. For example, the accumulation memory 140 accumulates an accumulated degree of annoyance, in which the degrees of annoyance with the first to the n-1-th snoring are accumulated.

The accumulating unit 150 accumulates the degrees of annoyance calculated by the calculating unit, and calculates the accumulated degree of annoyance. For example, when receiving the degree of annoyance with the n-th snoring from the calculating unit 130, the accumulating unit 150 acquires the accumulated degrees of annoyance with the first to the n-1-th snoring from the accumulation memory 140. Then, the calculating unit 130 calculates the accumulated degree of annoyance with the first to the n-th snoring by using, for example, Equation (3) below.

$$Annoy_total(n) = Annoy_total(n-1) + Annoy(n) \qquad (3)$$

In Equation (3), n represents the number of times of snoring. Annoy_total(n) represents the accumulated degree of annoyance with the first to the n-th snoring. Annoy_total(n-1) represents the accumulated degree of annoyance with the first to the n-1-th snoring. Annoy(n) represents the degree of annoyance with the n-th snoring. Namely, the accumulating unit 150 adds the degree of annoyance with the n-th snoring and the accumulated degree of annoyance with the first to the n-1-th snoring, to thereby calculate the accumulated degree of annoyance with the first to the n-th snoring.

FIG. 13 is a diagram illustrating an example of an accumulation result obtained by the accumulating unit. In FIG. 13, the vertical axis represents the accumulated degree of annoyance and the horizontal axis represents time. Values on the horizontal axis represent the number of times of snoring. As illustrated in FIG. 13, the accumulating unit 150 adds the degree of annoyance with the n-th snoring and the accumulated degree of annoyance with the first to the n-1-th snoring every time the accumulating unit 150 receives the degree of annoyance with the n-th snoring from the calculating unit 130. Then, the accumulating unit 150 outputs the accumulated degree of annoyance with the first to the n-th snoring to the determining unit 160. Furthermore, the accumulating unit 150 stores the accumulated degree of annoyance with the first to the n-th snoring in the accumulation memory 140. The accumulated degree of annoyance is an example of an accumulated effective volume.

The determining unit 160 determines whether the accumulated degree of annoyance accumulated by the accumulating unit 150 reaches a threshold. For example, the determining unit 160 determines whether the received accumulated degree of annoyance reaches the threshold every time the determining unit 160 receives the accumulated degree of annoyance with the first to the n-th snoring from the accumulating unit 150. When determining that the accumulated degree of annoyance reaches the threshold, the determining unit 160 outputs information indicating that the accumulated degree of annoyance has reached the threshold to a speaker or a monitor. On the other hand, when determining that the accumulated degree of annoyance has not reached the threshold, the determining unit 160 waits to receive a next accumulated degree of annoyance from the accumulating unit 150.

The flow of a process performed by the acoustic signal processing apparatus 100 according to the first embodiment will be explained below. FIG. 14 is a flowchart of the flow of the process performed by the acoustic signal processing apparatus according to the first embodiment. The process illustrated in FIG. 14 is executed upon detection of snoring by the detecting unit 120 for example.

As illustrated in FIG. 14, when the detecting unit 120 detects snoring (YES at Step S101), the calculating unit 130 calculates the degree of annoyance with the snoring detected by the detecting unit 120 based on the sound input signal (Step S102). The accumulating unit 150 accumulates the degrees of annoyance calculated by the calculating unit 130 and calculates an accumulated degree of annoyance (Step S103). The determining unit 160 determines whether the accumulated degree of annoyance accumulated by the accumulating unit 150 has reached the threshold (Step S104). When determining that the accumulated degree of annoyance has reached the threshold (YES at Step S104), the determining unit 160 outputs information indicating that the accumulated degree of annoyance has reached the threshold to a speaker or a monitor (Step S105). On the other hand, when determining that the accumulated degree of annoyance has not reached the threshold (NO at Step S104), the determining unit 160 returns the process to Step S101.

Advantageous effects of the acoustic signal processing apparatus 100 according to the first embodiment will be explained below. As described above, the acoustic signal processing apparatus 100 detects user's snoring contained in the sound input signal, and calculates the degree of annoyance with the detected snoring based on the sound input signal. Then, the acoustic signal processing apparatus 100 calculates the accumulated degree of annoyance by accumulating the calculated degrees of annoyance, and determines whether the calculated accumulated degree of annoyance has reached the threshold. Therefore, the acoustic signal processing apparatus 100 can accurately determine an acoustic signal generated by a human body. For example, even when the volume of snoring fluctuates, the acoustic signal processing apparatus 100 can prevent a detection failure or a detection delay when detecting annoying snoring, and can accurately determine the annoying snoring.

Furthermore, when the volume of snoring is equal to or greater than the threshold TH1 and smaller than the threshold TH2, the acoustic signal processing apparatus 100 calculates, as the degree of annoyance, a value proportional to the volume. Moreover, when the volume of snoring is smaller than the threshold TH1, the acoustic signal processing apparatus 100 calculates a predetermined minimum value as the degree of annoyance. Furthermore, when the volume of snoring is equal to or greater than the threshold TH2, the acoustic signal processing apparatus 100 calculates a predetermined maximum value as the degree of annoyance. Therefore, the acoustic signal processing apparatus 100 can accurately determine the annoying snoring in consideration of the degree to which other person feels annoyed with user's snoring.

Meanwhile, the configuration of the acoustic signal processing apparatus 100 illustrated in FIG. 1 is one example. Therefore, the acoustic signal processing apparatus 100 does not necessarily have to include all of the processing units illustrated in FIG. 1. For example, it is sufficient that the acoustic signal processing apparatus 100 includes the detecting unit, the calculating unit, the accumulating unit, and the determining unit.

Specifically, the detecting unit detects an acoustic signal that is generated by a human body and that is contained in the sound input signal. The calculating unit calculates an effective volume indicating the effective volume of the acoustic signal detected by the detecting unit based on the input signal. The accumulating unit calculates the accumulated effective volume by accumulating the effective volumes calculated by the calculating unit. The determining unit determines whether the accumulated effective volume calculated by the accumulating unit has reached a predetermined threshold.

## Second Embodiment

A mobile terminal according to a second embodiment will be explained. In the second embodiment, a case is explained that the mobile terminal determines annoying snoring. FIG. 15 is a block diagram illustrating an example of a configuration of the mobile terminal according to the second embodiment, As illustrated in FIG. 15, a mobile terminal 200 includes a sound collecting unit 210, an acoustic signal processing circuit 220, and an operation executing unit 230. The mobile terminal 200 is one example of the acoustic signal processing apparatus.

The sound collecting unit 210 collects a sound input signal. For example, the sound collecting unit 210 collects a sound input signal of sound generated near a sleeping user. The sound collecting unit 210 divides the collected sound input signal into a plurality of frames with a predetermined sample size and generates input signal data. The sound collecting unit 210 outputs the input signal data to the acoustic signal processing circuit 220. The sound collecting unit 210 corresponds to a sound collecting device, such as a microphone.

The acoustic signal processing circuit 220 detects snoring of the user from the sound input signal, and calculates the degree of annoyance according to the detected volume of snoring. The acoustic signal processing circuit 220 accumulates the degree of annoyance at every detection of user's snoring, and determines whether the accumulated degree of annoyance has reached a threshold. For example, the acoustic signal processing circuit 220 receives the input signal data from the sound collecting unit 210, and detects user's snoring from the input signal data. The acoustic signal processing circuit 220 calculates the degree of annoyance according to the detected volume of snoring, and accumulates the calculated degrees of annoyance. The acoustic signal processing circuit 220 determines that the user's snoring is annoying when the accumulated degree of annoyance reaches the threshold.

As illustrated in FIG. 15, the acoustic signal processing circuit 22C includes an input memory 221, a detecting unit 222, a calculating unit 223, an accumulation memory 224, an accumulating unit 225, and a determining unit 226. The explanation of the input memory 221, the detecting unit 222, the calculating unit 223, the accumulation memory 224, the accumulating unit 225, and the determining unit 226 illustrated in FIG. 15 is the same as the explanation of the input memory 110, the detecting unit 120, the calculating unit 130, the accumulation memory 140, the accumulating unit 150, and the determining unit 160 illustrated in FIG. 1.

The operation executing unit 230 executes a predetermined operation when the determining unit 226 determines that the accumulated degree of annoyance has reached the threshold. The operation executing unit 230 corresponds to, for example, a vibrating device or a speaker. For example, the operation executing unit 230 vibrates in a predetermined pattern or outputs sound with a predetermined volume when the user's snoring is determined as annoying.

As described above, the mobile terminal 200 according to the second embodiment can wake the user up by executing a predetermined operation to cause the user to stop snoring when the mobile terminal 200 determines that the accumulated degree of annoyance has reached the threshold.

Third Embodiment

An acoustic signal processing apparatus according to a third embodiment will be explained. The acoustic signal processing apparatus according to the third embodiment includes, similarly to the acoustic signal processing apparatus 100 illustrated in FIG. 1, the input memory 110, the detecting unit 120, the calculating unit 130, the accumulation memory 140, the accumulating unit 150, and the determining unit 160. Of these units, the explanations of the input memory 110, the detecting unit 120, the accumulation memory 140, the accumulating unit 150, and the determining unit 160 are the same as the explanation of the input memory 110, the detecting unit 120, the accumulation memory 14C, the accumulating unit 150, and the determining unit 160 illustrated in FIG. 1.

The calculating unit 130 has the same functions as those of the calculating unit 130 illustrated in FIG. 1. As the method for calculating the degree of annoyance, while the method using the above Equation (1) has been explained, the method is not limited to this example. For example, the calculating unit 130 may calculate the degree of annoyance caused by snoring by using Equation (4) below.

$$\mathrm{Annoy}(n) = \mathrm{fpow}(n) \times \mathrm{COEFF} \qquad (4)$$

In Equation (4), n represents the number of times of snoring. Annoy(n) represents the degree of annoyance with the n-th snoring. fpow(n) represents the volume of n-th snoring. COEFF represents an arbitrary constant.

The method for calculating the degree of annoyance using Equation (4) by the calculating unit 130 will be explained below. FIG. 16 is a diagram for explaining the method for calculating the degree of annoyance. In FIG. 16, the vertical axis represents the degree of annoyance and the horizontal axis represents a volume (dBA). As illustrated in FIG. 16, for example, the calculating unit 130 calculates, as the degree of annoyance Annoy(n), a value proportional to the volume fpow(n). Then, the calculating unit 130 outputs the calculated degree of annoyance to the accumulating unit 150 every time the calculating unit 130 calculates the degree of annoyance with the n-th snoring.

As described above, the acoustic signal. processing apparatus 100 according to the third embodiment calculates a value proportional to the volume of snoring as the degree of annoyance with the snoring. Therefore, the acoustic signal processing apparatus 100 can accurately determine annoying snoring with low processing load.

Fourth Embodiment

An acoustic signal processing apparatus according to a fourth embodiment will be explained. The acoustic signal processing apparatus according to the fourth embodiment reduces the accumulated degree of annoyance when a predetermined minimum value is continuously calculated as the degree of annoyance for a predetermined time. This takes advantage of the fact that, even when the degrees of annoyance are accumulated, snoring becomes less annoying to other people when the snoring with a low volume continues for a predetermined time. The acoustic signal processing apparatus according to the fourth embodiment includes the input memory 110, the detecting unit 120, the calculating unit 130, the accumulation memory 140, the accumulating unit 150, and the determining unit 160 similarly to the acoustic signal processing apparatus 100 illustrated in FIG. 1. Of these units, the explanation of the input memory 110, the detecting unit 120, the calculating unit 130, the accumulation memory 140, and the determining unit 160 is the same as the explanation of the input memory 110, the detecting unit 120, the calculating unit 130, the accumulation memory 140, and the determining unit 160 illustrated in FIG. 1.

The accumulating unit 150 has the same functions as those of the accumulating unit 150 illustrated in FIG. 1. The accumulating unit 150 reduces the accumulated degree of annoyance when the calculating unit 130 continuously calculates a predetermined minimum value as the degree of annoyance for a predetermined time. For example, the accumulating unit 150 calculates the degree of annoyance with snoring by using Equation (5) below.

$$\mathrm{Annoy_total}(n) = \begin{cases} 0 & (\mathrm{Annoy}(n) = \mathrm{Annoy}(n-1) = \ldots = \mathrm{Annoy}(n-N) = 0) \\ \mathrm{Annoy_total}(n-1) + \mathrm{Annoy}(n) & (otherwise) \end{cases} \quad (5)$$

In Equation (5), n represents the number of times of snoring. Annoy_total(n; represents the accumulated degree of annoyance with the first to the n-th snoring. Annoy_total(n-1) represents the accumulated degree of annoyance with the first to the n-1-th snoring. Annoy(n) represents the degree of annoyance with the n-th snoring. N represents the number of times of snoring contained in a predetermined time that is determined in advance to reduce the accumulated degree of annoyance. Namely, when all the degrees of annoyance with snoring received from the n-N-th time to the n-th time are "0", the accumulating unit 150 reduces the accumulated degree of annoyance with the first to the n-th snoring to "0". On the other hand, in other cases, the accumulating unit 150 adds the degree of annoyance with the n-th snoring and the accumulated degree of annoyance with the first to the n-1-th snoring to calculate the accumulated degree of annoyance with the first to the n-th snoring. Then, the accumulating unit 150 outputs the accumulated degree of annoyance with the first to the n-th snoring to the determining unit 160. Furthermore, the accumulating unit 150 stores the accumulated degree of annoyance with the first to the n-th snoring in the accumulation memory 140. While a case has been explained that the accumulated degree of annoyance is reduced to "0", it is not limited thereto. For example, the accumulating unit 150 may reduce the accumulated degree of annoyance to half.

As described above, the acoustic signal processing apparatus 100 according to the fourth embodiment reduces the accumulated degree of annoyance when the calculating unit 130 continuously detects the predetermined minimum value as the degree of annoyance for a predetermined time. Therefore, the acoustic signal processing apparatus 100 can accurately determine the annoying snoring in consideration of the fact that snoring becomes less annoying to other people when the snoring with a low volume continues for a predetermined time.

Fifth Embodiment

While the embodiments of the present invention have been described above, the present invention may be embodied in various forms other than the embodiments described above. Therefore, other embodiments will be described below.

For example, in the embodiment described above, it is explained that the acoustic signal processing apparatus 100 and the mobile terminal 200 detects an acoustic signal generated by a human body as snoring; however, the present invention is not limited thereto. For example, the acoustic signal processing apparatus 100 and the mobile terminal 200 may detect other acoustic signals, such as teeth grinding or monology, as the acoustic signal generated by a human body. For another example, the acoustic signal processing apparatus 100 and the mobile terminal 200 may detect an acoustic signal in a frequency band that is annoying to other people. In this way, as a technology for detecting the acoustic signal generated by a human body, a well-known technology, such as a technology disclosed in Japanese Laid-open Patent Publication No. 7-184948 or a technology disclosed in Japanese Laid-open Patent Publication No. 2004-187961, may arbitrarily be selected and used.

Moreover, the components of the acoustic signal processing apparatus 100 and the mobile terminal 200 illustrated in FIG. 1 and 15 are functionally conceptual and do not necessarily have to be physically configured in the manner illustrated in the drawings. Namely, specific forms of distribution and integration of the acoustic signal processing apparatus 100 and the mobile terminal 200 are not limited to those illustrated in the drawings, and all or part of the acoustic signal processing apparatus 100 and the mobile terminal 200 can be functionally or physically distributed or integrated in arbitrary units according to various loads and the state of use. For example, it may be possible to integrate the calculating unit 130 and the accumulating unit 150 illustrated in FIG. 1.

Furthermore, the processing functions implemented by the detecting units 120 and 222, the calculating units 130 and 223, the accumulating units 150 and 225, and the determining units 160 and 226 are realized as described below. Specifically, all or an arbitrary part of the processing functions may be realized by a CPU (Central Processing Unit) and a program analyzed and executed by the CPU, or may be realized by hardware using wired logic.

Moreover, the input memories 110 and 221 and the accumulation memories 140 and 224 correspond to a semiconductor memory device, such as a RAM (Random Access Memory), a ROM (Read Only Memory), or a flash memory (Flash Memory), or corresponds to a storage device, such as a hard disk or an optical disk.

The acoustic signal processing apparatus 100 and the mobile terminal 200 may be realized by mounting the functions of the acoustic signal processing apparatus 100 and the mobile terminal 200 onto a known information processing apparatus. The known information processing apparatus corresponds to a device, such as a personal computer, a workstation, a mobile phone, a PHS (Personal Handy-phone System) terminal, a mobile communication terminal, or a PDA (Personal Digital Assistant).

FIG. 17 is a diagram illustrating an example of a computer that executes an acoustic signal processing program. As illustrated in FIG. 17, a computer 300 includes a CPU 301 that executes various types of arithmetic processing, an input device 302 that receives input of data from a user, and a monitor 303. The computer 300 also includes a medium reading device 304 that reads a program or the like from a storage medium, and an interface device 305 that transmits and receives data to and from other devices. The computer 300 also includes a RAM (Random Access Memory) 306 for temporarily storing various types of data, and a hard disk device 307. The devices 301 to 307 are connected to a bus 308.

The hard disk device 307 stores therein an acoustic signal processing program 307a that has the same functions as those of the processing units of the detecting units 120 and 222, the calculating units 130 and 223, the accumulating units 150 and 225, and the determining units 160 and 226 illustrated in FIG. 1 and 15. The hard disk device 307 also stores therein various types of data 307b for realizing the acoustic signal processing program 307a.

The CPU 301 reads the acoustic signal processing program 307a from the hard disk device 3C7 and loads and executes it on the RAM 306, so that the acoustic signal processing program 307a functions as an acoustic signal processing process 306a. Namely, the acoustic signal processing program 307a functions as a process that is the same as the processing units of the detecting units 120 and 222, the calculating units 130 and 223, the accumulating units 150 and 225, and the determining units 160 and 226.

The acoustic signal processing program 307a described above does not necessarily have to be stored in the hard disk device 307. For example, the computer 300 may read the program stored in a computer-readable recording medium and execute the read program. The computer-readable recording medium corresponds to, for example, a portable recording medium, such as a CD-ROM, a DVD disk, or a USB memory; a semiconductor memory, such as a flash memory; or a hard disk drive. It may also be possible to store the program in a device connected to a public line, the Internet, a LAN (Local Area Network), or a WAN (Wide Area Network), and cause the computer 300 to read the program from the device and execute the read program.

[Explanation of Reference]

100 ACOUSTIC SIGNAL PROCESSING APPARATUS
110 INPUT MEMORY
120 DETECTING UNIT
121 FFT UNIT
122 POWER SPECTRUM CALCULATING UNIT
123 SIMILARITY CALCULATING UNIT
124 DURATION CALCULATING UNIT
125 SNORING DETERMINING UNIT
130 CALCULATING UNIT
140 ACCUMULATION MEMORY
150 ACCUMULATING UNIT
160 DETERMINING UNIT
200 MOBILE TERMINAL
210 SOUND COLLECTING UNIT
220 ACOUSTIC SIGNAL PROCESSING CIRCUIT
221 INPUT MEMORY
222 DETECTING UNIT
223 CALCULATING UNIT
224 ACCUMULATION MEMORY
225 ACCUMULATING UNIT
226 DETERMINING UNIT

| | |
|---|---|
| 230 | OPERATION EXECUTING UNIT |
| 300 | COMPUTER |
| 301 | CPU |
| 302 | INPUT DEVICE |
| 303 | MONITOR |
| 304 | MEDIUM READING DEVICE |
| 305 | INTERFACE DEVICE |
| 306a | ACOUSTIC SIGNAL PROCESSING PROCESS |
| 307 | HARD DISK DEVICE |
| 307a | ACOUSTIC SIGNAL PROCESSING PROGRAM |
| 307b | VARIOUS TYPES OF DATA. |
| 308 | BUS |

## Claims

**1.** An acoustic signal processing apparatus (100, 220) comprising:

a detecting unit (120, 222) that is configured to detect an acoustic signal that corresponds to an interval containing snoring in a sound input signal;
a calculating unit (130, 223) that is configured to calculate an effective volume of the acoustic signal detected by the detecting unit (120, 222), based on the input signal;
an accumulating unit (150, 225) that is configured to accumulate the effective volume calculated by the calculating unit (130, 223) to calculate an accumulated effective volume; and
a determining unit (160, 226) that is configured to determine whether the accumulated effective volume calculated by the accumulating unit (150, 225) has reached a predetermined threshold, wherein
when a level of the input signal is equal to or greater than a first threshold and smaller than a second threshold, the calculating unit (130, 223) is configured to calculate a value proportional to the level as the effective volume,
when the level is smaller than the first threshold, the calculating unit (130, 223) is configured to calculate a predetermined minimum value as the effective volume, and
when the level is equal to or greater than the second threshold, the calculating unit (130, 223) is configured to calculate a predetermined maximum value as the effective volume.

**2.** The acoustic signal processing apparatus (100, 220) according to claim 1, wherein the accumulating unit (150, 225) is configured to reduce the accumulated effective value when the calculating unit (130, 223) continues to calculate a predetermined minimum value as the effective volume for a predetermined time.

**3.** The acoustic signal processing apparatus (100, 220) according to claim 1 or 2, further comprising an operation executing unit (230) that is configured to execute a predetermined operation when the determining unit (160, 226) determines that the accumulated effective volume has reached the threshold.

**4.** An acoustic signal processing method executed by a computer, the acoustic signal processing method comprising:

detecting an acoustic signal that corresponds to an interval containing snoring in a sound input signal;
calculating an effective volume of the acoustic signal detected at the detecting the acoustic signal, based on the input signal;
calculating an accumulated effective volume by accumulating effective volume calculated at the calculating the effective volume; and
determining whether the accumulated effective volume calculated at the calculating the accumulated effective volume has reached a predetermined threshold, wherein
the calculating the effective volume includes:

calculating, as the effective volume, a value proportional to the effective volume when a level of the input signal is equal to or greater than a first threshold and smaller than a second threshold;
calculating, as the effective volume, a predetermined minimum value when the level is smaller than the first threshold; and
calculating, as the effective volume, a predetermined maximum value when the level is equal to or greater than the second threshold.

5. The acoustic signal processing method according to claim 4, wherein the calculating the accumulated effective volume includes reducing the accumulated effective volume when a predetermined minimum value is continuously calculated as the effective volume for a predetermined time at the calculating the effective volume.

6. The acoustic signal processing method according to claim 4 or 5, further comprising executing a predetermined operation when it is determined that the accumulated effective volume has reached the threshold at the determining whether the accumulated effective volume has reached the threshold.

7. An acoustic signal processing program that, when executed, causes a computer to execute:

   detecting an acoustic signal that corresponds to an interval containing snoring in a sound input signal;
   calculating an effective volume of the acoustic signal detected at the detecting the acoustic signal, based on the input signal;
   calculating an accumulated effective volume by accumulating effective volume calculated at the calculating the effective volume; and
   determining whether the accumulated effective volume calculated at the calculating the accumulated effective volume has reached a predetermined threshold, wherein
   the calculating the effective volume includes:

      calculating, as the effective volume, a value proportional to the effective volume when a level of the input signal is equal to or greater than a first threshold and smaller than a second threshold;
      calculating, as the effective volume, a predetermined minimum value when the level is smaller than the first threshold; and
      calculating, as the effective volume, a predetermined maximum value when the level is equal to or greater than the second threshold.

8. The acoustic signal processing program according to claim 7, wherein the calculating the accumulated effective volume includes reducing the accumulated effective volume when a predetermined minimum value is continuously calculated as the effective volume for a predetermined time at the calculating the effective volume.

9. The acoustic signal processing program according to claim 7 or 8, further comprising executing a predetermined operation when it is determined that the accumulated effective volume has reached the threshold at the determining whether the accumulated effective volume has reached the threshold.

## Patentansprüche

1. Vorrichtung (100, 220) zum Verarbeiten akustischer Signale, umfassend:

   eine Detektiereinheit (120, 222), die konfiguriert ist, um ein akustisches Signal zu detektieren, das einem Intervall entspricht, das Schnarchen in einem Toneingangssignal enthält;
   eine Berechnungseinheit (130, 223), die konfiguriert ist, um eine effektive Lautstärke des durch die Detektiereinheit (120, 222) detektierten akustischen Signals basierend auf dem Eingangssignal zu berechnen;
   eine Akkumuliereinheit (150, 225), die konfiguriert ist, um die effektive Lautstärke zu akkumulieren, die durch die Berechnungseinheit (130, 223) berechnet wird, um eine akkumulierte effektive Lautstärke zu berechnen; und
   eine Bestimmungseinheit (160, 226), die konfiguriert ist, um zu bestimmen, ob die durch die Akkumuliereinheit (150, 225) berechnete akkumulierte effektive Lautstärke eine vorbestimmte Schwelle erreicht hat, wobei
   wenn ein Pegel des Eingangssignals gleich oder größer als eine erste Schwelle, und geringer als eine zweite Schelle ist, die Berechnungseinheit (130, 223) konfiguriert ist, um einen dem Pegel proportionalen Wert als die effektive Lautstärke zu berechnen,
   wenn der Pegel geringer als die erste Schwelle ist, die Berechnungseinheit (130, 223) konfiguriert ist, um einen vorbestimmten minimalen Wert als die effektive Lautstärke zu berechnen, und
   wenn der Pegel gleich oder größer als die zweite Schwelle ist, die Berechnungseinheit (130, 223) konfiguriert ist, um einen vorbestimmten maximalen Wert als die effektive Lautstärke zu berechnen.

2. Vorrichtung (100, 220) zum Verarbeiten akustischer Signale nach Anspruch 1, wobei die Akkumuliereinheit (150, 225) konfiguriert ist, um den akkumulierten effektiven Wert zu reduzieren, wenn die Berechnungseinheit (130, 223) fortfährt, einen vorbestimmten minimalen Wert als die effektive Lautstärke für eine vorbestimmte Zeit zu berechnen.

3. Vorrichtung (100, 220) zum Verarbeiten akustischer Signale nach Anspruch 1 oder 2, ferner eine Operation ausführende Einheit (230) umfassend, die konfiguriert ist, um eine vorbestimmte Operation auszuführen, wenn die Bestimmungseinheit (160, 226) bestimmt, dass die akkumulierte effektive Lautstärke die Schwelle erreicht hat.

4. Verfahren zum Verarbeiten akustischer Signale, das von einem Computer ausgeführt wird, wobei das Verfahren zum Verarbeiten akustischer Signale umfasst:

Detektieren eines akustischen Signals, das einem Intervall entspricht, das Schnarchen in einem Toneingangssignal enthält;
Berechnen einer effektiven Lautstärke des akustischen Signals, das beim Detektieren des akustischen Signals detektiert wird, basierend auf dem Eingangssignal;
Berechnen einer akkumulierten effektiven Lautstärke, indem eine effektive Lautstärke akkumuliert wird, die beim Berechnen der effektiven Lautstärke berechnet wird; und
Bestimmen, ob die akkumulierte effektive Lautstärke, die beim Berechnen der akkumulierten effektiven Lautstärke berechnet wird, eine vorbestimmte Schwelle erreicht hat, wobei
das Berechnen der effektiven Lautstärke einschließt:

Berechnen, als die effektive Lautstärke, eines zur effektiven Lautstärke proportionalen Wertes, wenn ein Pegel des Eingangssignals gleich oder größer als eine erste Schwelle und geringer als eine zweite Schwelle ist;
Berechnen, als die effektive Lautstärke, eines vorbestimmten minimalen Wertes, wenn der Pegel geringer als die erste Schwelle ist; und
Berechnen, als die effektive Lautstärke, eines vorbestimmten maximalen Wertes, wenn der Pegel gleich oder größer als die zweite Schwelle ist.

5. Verfahren zum Verarbeiten akustischer Signale nach Anspruch 4, wobei das Berechnen der akkumulierten effektiven Lautstärke ein Reduzieren der akkumulierten effektiven Lautstärke einschließt, wenn beim Berechnen der effektiven Lautstärke kontinuierlich ein vorbestimmter minimaler Wert als die effektive Lautstärke für eine vorbestimmte Zeit berechnet wird.

6. Verfahren zum Verarbeiten akustischer Signale nach Anspruch 4 oder 5, ferner ein Ausführen einer vorbestimmten Operation umfassend, wenn bestimmt wird, dass beim Bestimmen, ob die akkumulierte effektive Lautstärke die Schwelle erreicht hat, die akkumulierte effektive Lautstärke die Schwelle erreicht hat.

7. Programm zum Verarbeiten akustischer Signale, das, wenn es ausgeführt wird, einen Computer veranlasst, auszuführen:

Detektieren eines akustischen Signals, das einem Intervall entspricht, das Schnarchen in einem Toneingangssignal enthält;
Berechnen einer effektiven Lautstärke des akustischen Signals, das beim Detektieren des akustischen Signals detektiert wird, basierend auf dem Eingangssignal;
Berechnen einer akkumulierten effektiven Lautstärke, indem eine effektive Lautstärke akkumuliert wird, die beim Berechnen der effektiven Lautstärke berechnet wird; und
Bestimmen, ob die akkumulierte effektive Lautstärke, die beim Berechnen der akkumulierten effektiven Lautstärke berechnet wird, eine vorbestimmte Schwelle erreicht hat, wobei
das Berechnen der effektiven Lautstärke einschließt:

Berechnen, als die effektive Lautstärke, eines zur effektiven Lautstärke proportionalen Wertes, wenn ein Pegel des Eingangssignals gleich oder größer als eine erste Schwelle und geringer als eine zweite Schwelle ist;
Berechnen, als die effektive Lautstärke, eines vorbestimmten minimalen Wertes, wenn der Pegel geringer als die erste Schwelle ist; und
Berechnen, als die effektive Lautstärke, eines vorbestimmten maximalen Wertes, wenn der Pegel gleich oder größer als die zweite Schwelle ist.

8. Programm zum Verarbeiten akustischer Signale nach Anspruch 7, wobei das Berechnen der akkumulierten effektiven Lautstärke ein Reduzieren der akkumulierten effektiven Lautstärke einschließt, wenn beim Berechnen der effektiven Lautstärke kontinuierlich ein vorbestimmter minimaler Wert als die effektive Lautstärke für eine vorbestimmte Zeit berechnet wird.

**9.** Programm zum Verarbeiten akustischer Signale nach Anspruch 7 oder 8, ferner ein Ausführen einer vorbestimmten Operation umfassend, wenn bestimmt wird, dass beim Bestimmen, ob die akkumulierte effektive Lautstärke die Schwelle erreicht hat die akkumulierte effektive Lautstärke die Schwelle erreicht hat.

**Revendications**

**1.** Dispositif de traitement de signal acoustique (100, 220) comprenant :

une unité de détection (120, 222) qui est configurée pour détecter un signal acoustique qui correspond à un intervalle contenant un ronflement dans un signal d'entrée sonore ;
une unité de calcul (130, 223) qui est configurée pour calculer un volume effectif du signal acoustique détecté par l'unité de détection (120, 222), sur la base du signal d'entrée ;
une unité d'accumulation (150, 225) qui est configurée pour accumuler le volume effectif calculé par l'unité de calcul (130, 223) pour calculer un volume effectif accumulé; et
une unité de détermination (160, 226) qui est configurée pour déterminer si le volume effectif accumulé calculé par l'unité d'accumulation (150, 225) a atteint un seuil prédéterminé, dans lequel
lorsqu'un niveau du signal d'entrée est supérieur ou égal à un premier seuil et inférieur à un second seuil, l'unité de calcul (130, 223) est configurée pour calculer une valeur proportionnelle au niveau comme volume effectif, lorsque le niveau est inférieur au premier seuil, l'unité de calcul (130, 223) est configurée pour calculer une valeur minimale prédéterminée comme volume effectif, et
lorsque le niveau est supérieur ou égal au second seuil, l'unité de calcul (130, 223) est configurée pour calculer une valeur maximale prédéterminée comme volume effectif.

**2.** Dispositif de traitement de signal acoustique (100, 220) selon la revendication 1, dans lequel l'unité d'accumulation (150, 225) est configurée pour réduire la valeur effective accumulée lorsque l'unité de calcul (130, 223) continue à calculer une valeur minimale prédéterminée comme volume effectif pendant une durée prédéterminée.

**3.** Dispositif de traitement de signal acoustique (100, 220) selon la revendication 1 ou 2, comprenant en outre une unité d'exécution d'opération (230) qui est configurée pour exécuter une opération prédéterminée lorsque l'unité de détermination (160, 226) détermine que le volume effectif accumulé a atteint le seuil.

**4.** Procédé de traitement de signal acoustique exécuté par un ordinateur, le procédé de traitement de signal acoustique comprenant :

la détection d'un signal acoustique qui correspond à un intervalle contenant un ronflement dans un signal d'entrée sonore ;
le calcul d'un volume effectif du signal acoustique détecté au moment de la détection du signal acoustique, sur la base du signal d'entrée ;
le calcul d'un volume effectif accumulé en accumulant le volume effectif calculé au moment du calcul du volume effectif ; et
le fait de déterminer si le volume effectif accumulé calculé au moment du calcul du volume effectif accumulé a atteint un seuil prédéterminé, dans lequel
le calcul du volume effectif comprend :

> le calcul, comme volume effectif, d'une valeur proportionnelle au volume effectif lorsqu'un niveau du signal d'entrée est supérieur ou égal à un premier seuil et inférieur à un second seuil ;
> le calcul, comme volume effectif, d'une valeur minimale prédéterminée lorsque le niveau est inférieur au premier seuil ; et
> le calcul, comme volume effectif, d'une valeur maximale prédéterminée lorsque le niveau est supérieur ou égal au second seuil.

**5.** Procédé de traitement de signal acoustique selon la revendication 4, dans lequel le calcul du volume effectif accumulé comprend la réduction du volume effectif accumulé lorsqu'une valeur minimale prédéterminée est calculée en continu comme volume effectif pendant une durée prédéterminée au moment du calcul du volume effectif.

**6.** Procédé de traitement de signal acoustique selon la revendication 4 ou 5, comprenant en outre l'exécution d'une opération prédéterminée lorsqu'il est déterminé que le volume effectif accumulé a atteint le seuil au moment de déterminer si le volume effectif accumulé a atteint le seuil.

**7.** Programme de traitement de signal acoustique qui, lorsqu'il est exécuté, amène un ordinateur à exécuter :

la détection d'un signal acoustique qui correspond à un intervalle contenant un ronflement dans un signal d'entrée sonore ;
le calcul d'un volume effectif du signal acoustique détecté au moment de la détection du signal acoustique, sur la base du signal d'entrée;
le calcul d'un volume effectif accumulé en accumulant le volume effectif calculé au moment du calcul du volume effectif; et
le fait de déterminer si le volume effectif accumulé calculé au moment du calcul du volume effectif accumulé a atteint un seuil prédéterminé, dans lequel
le calcul du volume effectif comprend :

le calcul, comme volume effectif, d'une valeur proportionnelle au volume effectif lorsqu'un niveau du signal d'entrée est supérieur ou égal à un premier seuil et inférieur à un second seuil ;
le calcul, comme volume effectif, d'une valeur minimale prédéterminée lorsque le niveau est inférieur au premier seuil ; et
le calcul, comme volume effectif, d'une valeur maximale prédéterminée lorsque le niveau est supérieur ou égal au second seuil.

**8.** Programme de traitement de signal acoustique selon la revendication 7, dans lequel le calcul du volume effectif accumulé comprend la réduction du volume effectif accumulé lorsqu'une valeur minimale prédéterminée est calculée en continu comme volume effectif pendant une durée prédéterminée au moment du calcul du volume effectif.

**9.** Programme de traitement de signal acoustique selon la revendication 7 ou 8, comprenant en outre l'exécution d'une opération prédéterminée lorsqu'il est déterminé que le volume effectif accumulé a atteint le seuil au moment de déterminer si le volume effectif accumulé a atteint le seuil.

FIG.1

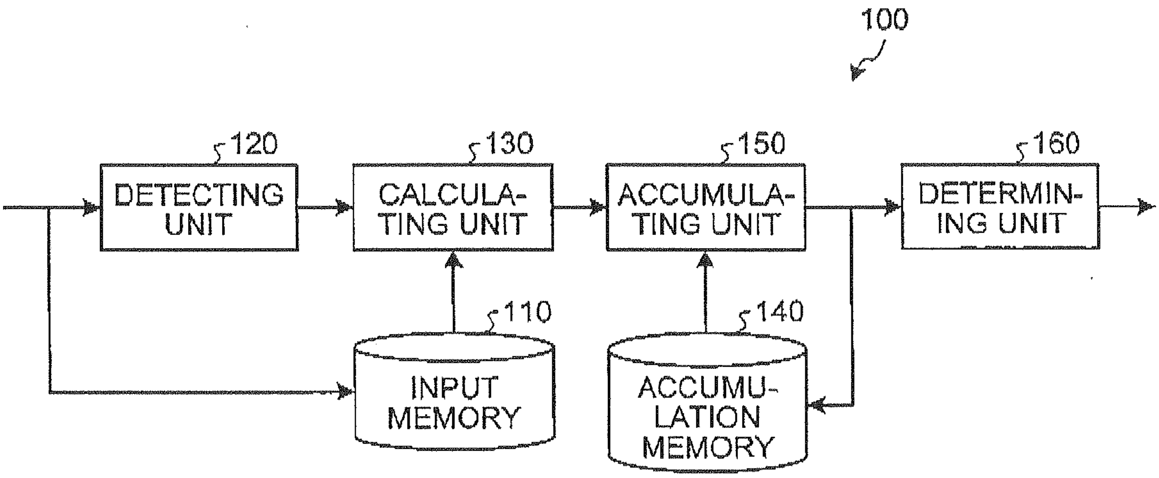
100
120
DETECTING UNIT
130
CALCULA-TING UNIT
150
ACCUMULA-TING UNIT
160
DETERMIN-ING UNIT
110
INPUT MEMORY
140
ACCUMU-LATION MEMORY

FIG.2

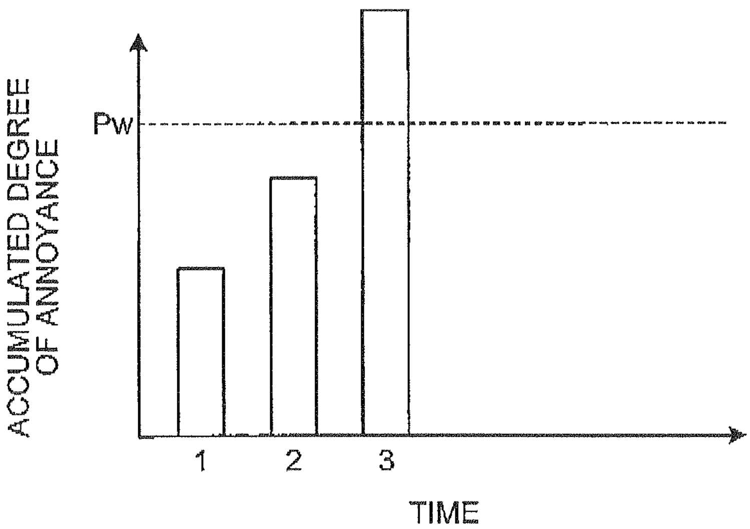
ACCUMULATED DEGREE OF ANNOYANCE
Pw
1
2
3
TIME

FIG.3

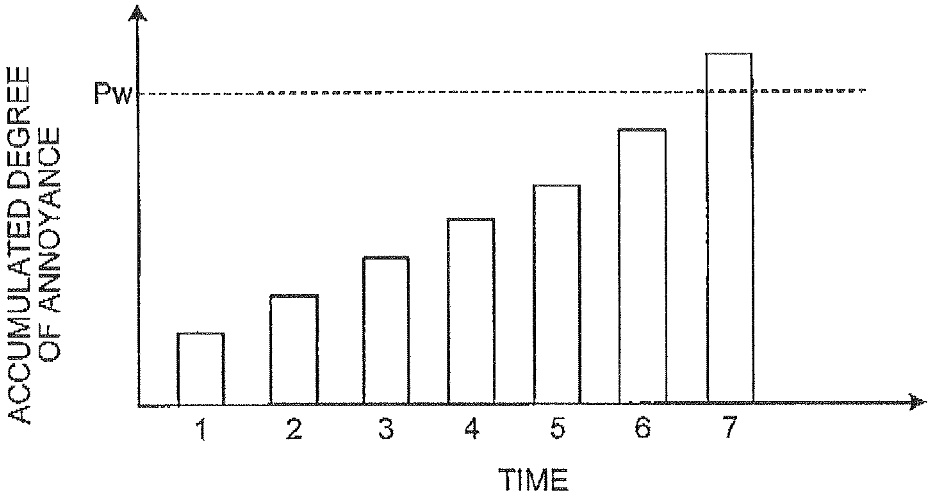
ACCUMULATED DEGREE
OF ANNOYANCE
Pw
1
2
3
4
5
6
7
TIME

FIG.4

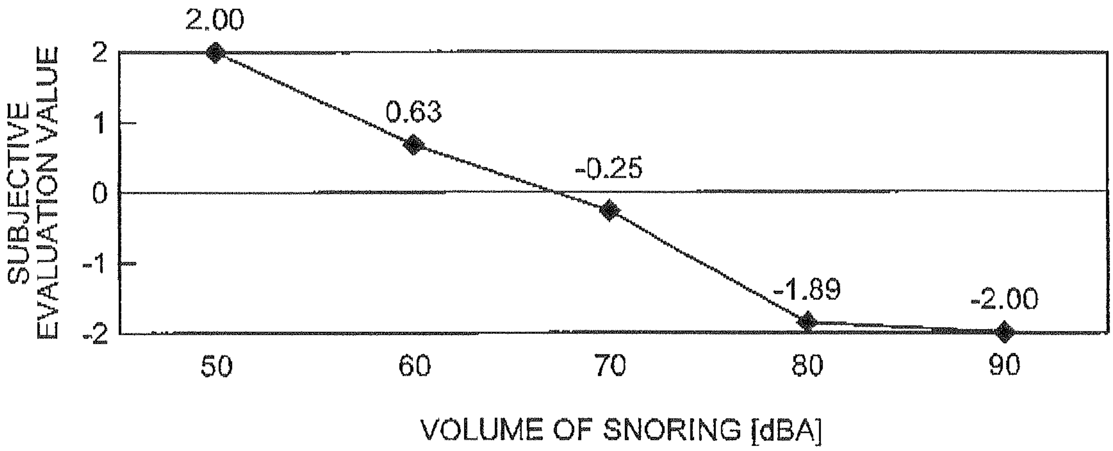
SUBJECTIVE
EVALUATION VALUE
2
1
0
-1
-2
2.00
0.63
-0.25
-1.89
-2.00
50
60
70
80
90
VOLUME OF SNORING [dBA]

FIG.5

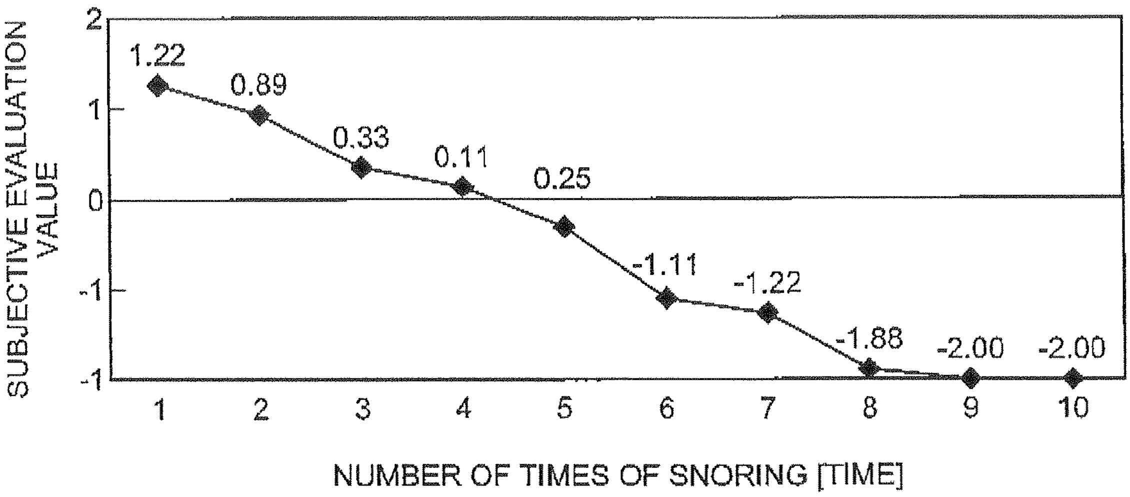
SUBJECTIVE EVALUATION VALUE
2
1
0
-1
-1
1.22
0.89
0.33
0.11
0.25
-1.11
-1.22
-1.88
-2.00
-2.00
1
2
3
4
5
6
7
8
9
10
NUMBER OF TIMES OF SNORING [TIME]

FIG.6

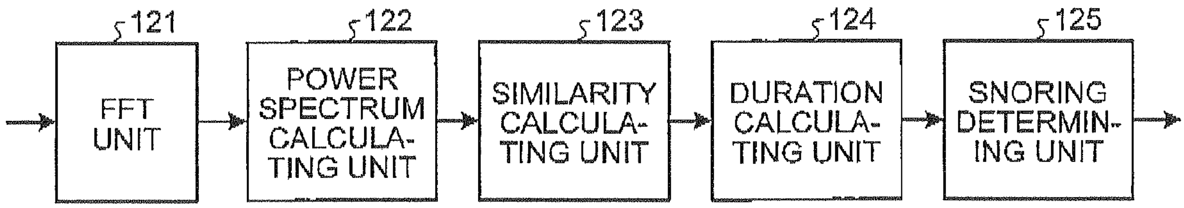
121
FFT UNIT
122
POWER SPECTRUM CALCULATING UNIT
123
SIMILARITY CALCULATING UNIT
124
DURATION CALCULATING UNIT
125
SNORING DETERMINING UNIT

FIG.7

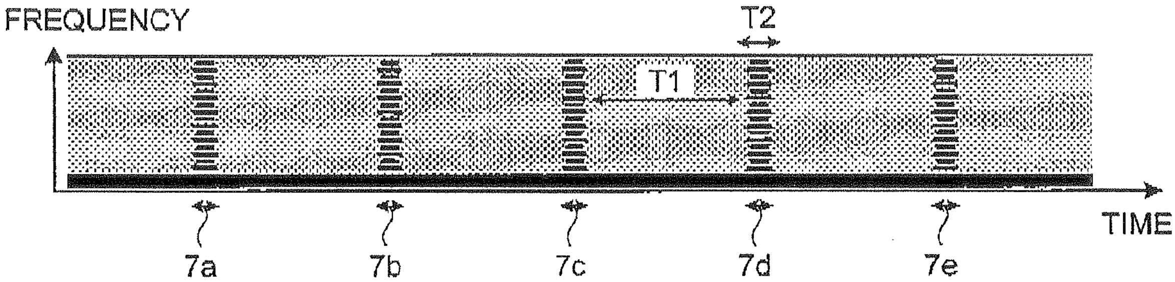
FREQUENCY
T2
T1
TIME
7a
7b
7c
7d
7e

FIG.8

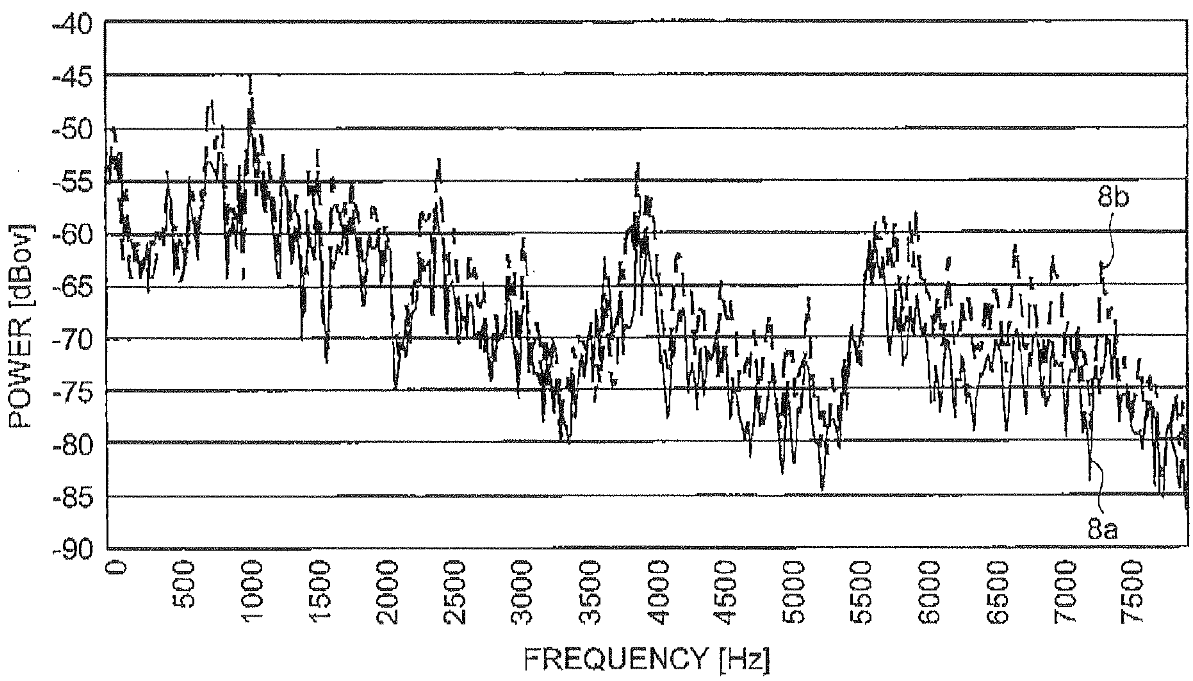
-40
-45
-50
-55
-60
-65
-70
-75
-80
-85
-90
POWER [dBov]
0
500
1000
1500
2000
2500
3000
3500
4000
4500
5000
5500
6000
6500
7000
7500
FREQUENCY [Hz]
8a
8b

FIG.9

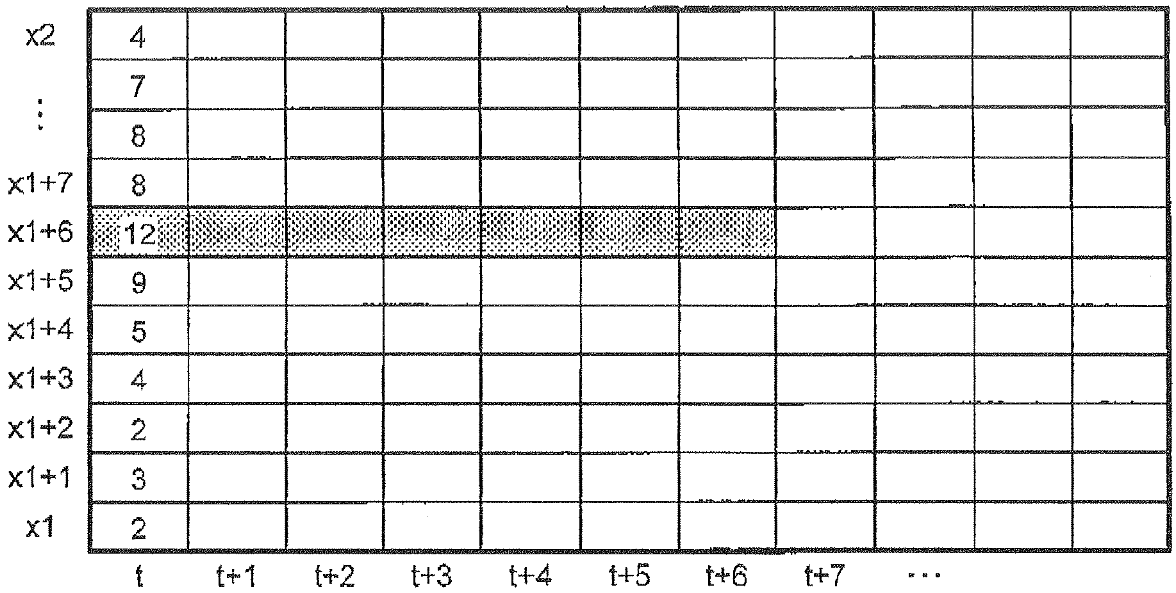
x2
⋮
x1+7
x1+6
x1+5
x1+4
x1+3
x1+2
x1+1
x1
4
7
8
8
12
9
5
4
2
3
2
t
t+1
t+2
t+3
t+4
t+5
t+6
t+7
...

FIG.10

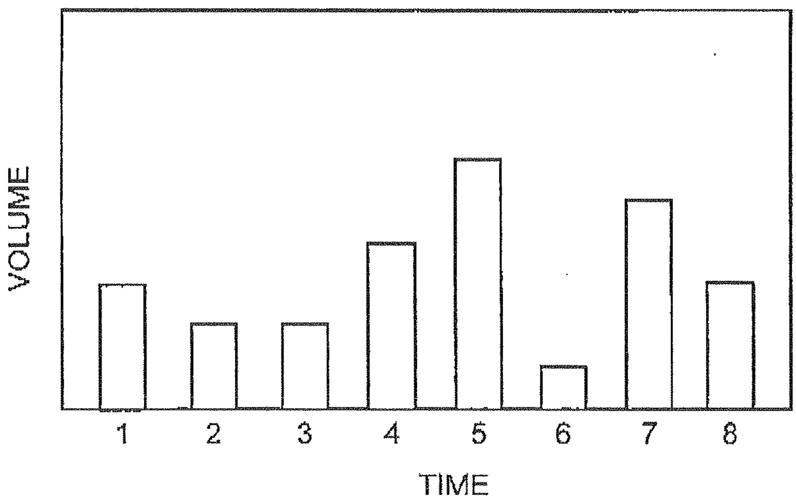
VOLUME
1
2
3
4
5
6
7
8
TIME

FIG.11

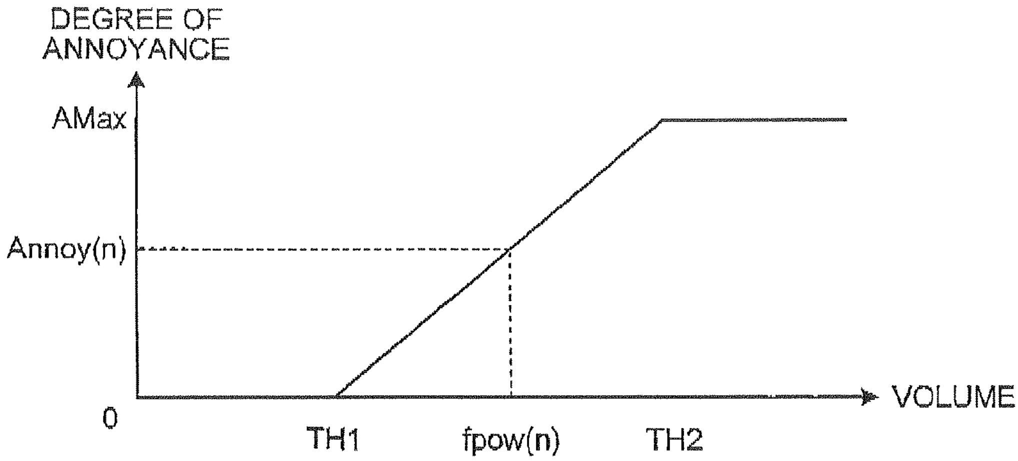
DEGREE OF ANNOYANCE
AMax
Annoy(n)
0
TH1
fpow(n)
TH2
VOLUME

FIG.12

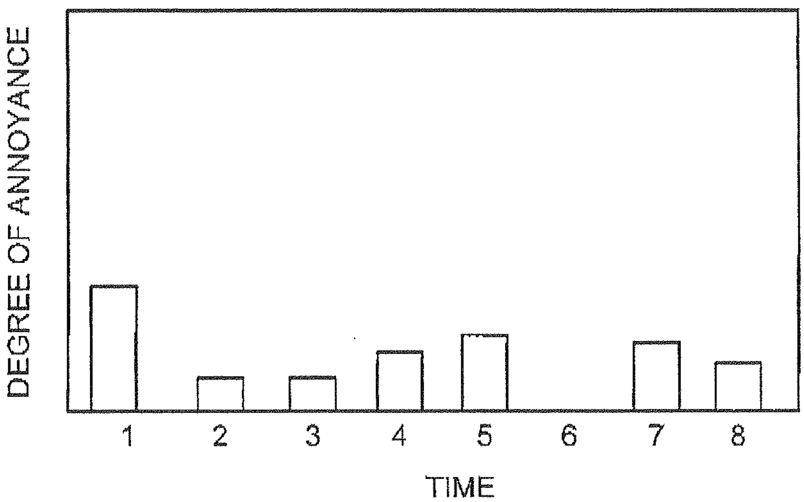
DEGREE OF ANNOYANCE
1
2
3
4
5
6
7
8
TIME

FIG.13

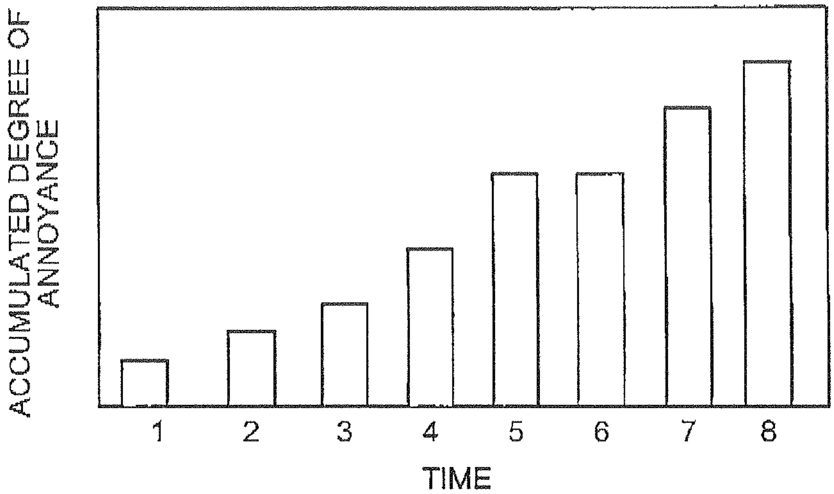
ACCUMULATED DEGREE OF ANNOYANCE
1
2
3
4
5
6
7
8
TIME

FIG.14

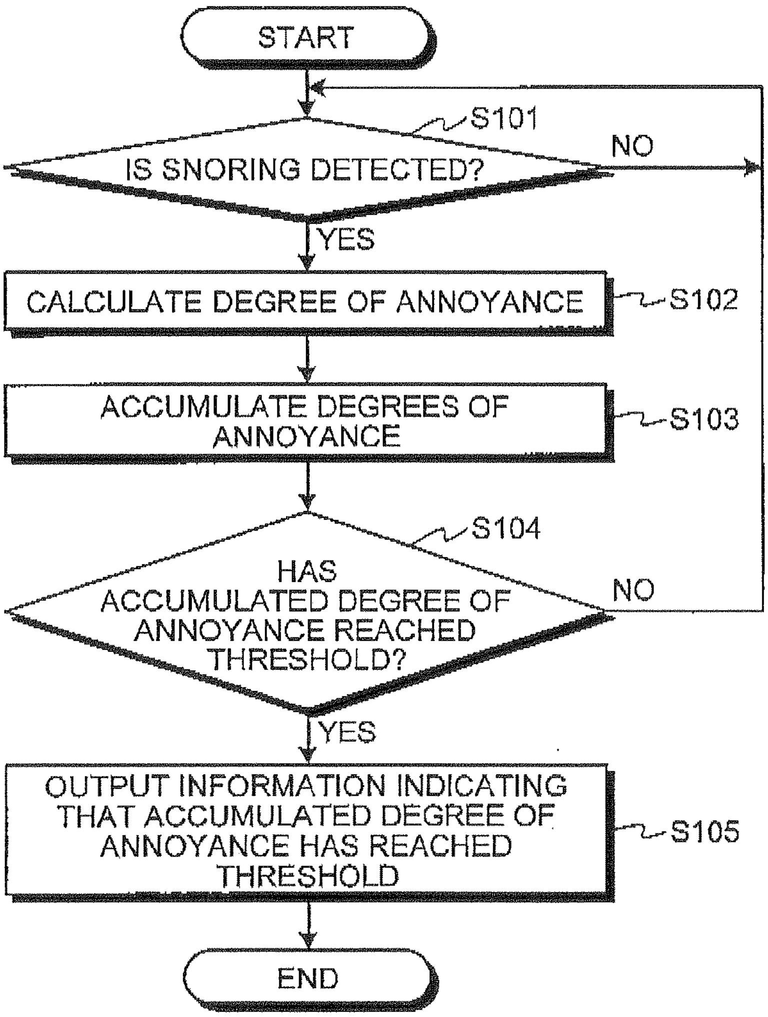
START
S101
IS SNORING DETECTED?
NO
YES
CALCULATE DEGREE OF ANNOYANCE
S102
ACCUMULATE DEGREES OF ANNOYANCE
S103
S104
HAS ACCUMULATED DEGREE OF ANNOYANCE REACHED THRESHOLD?
NO
YES
OUTPUT INFORMATION INDICATING THAT ACCUMULATED DEGREE OF ANNOYANCE HAS REACHED THRESHOLD
S105
END

FIG.15

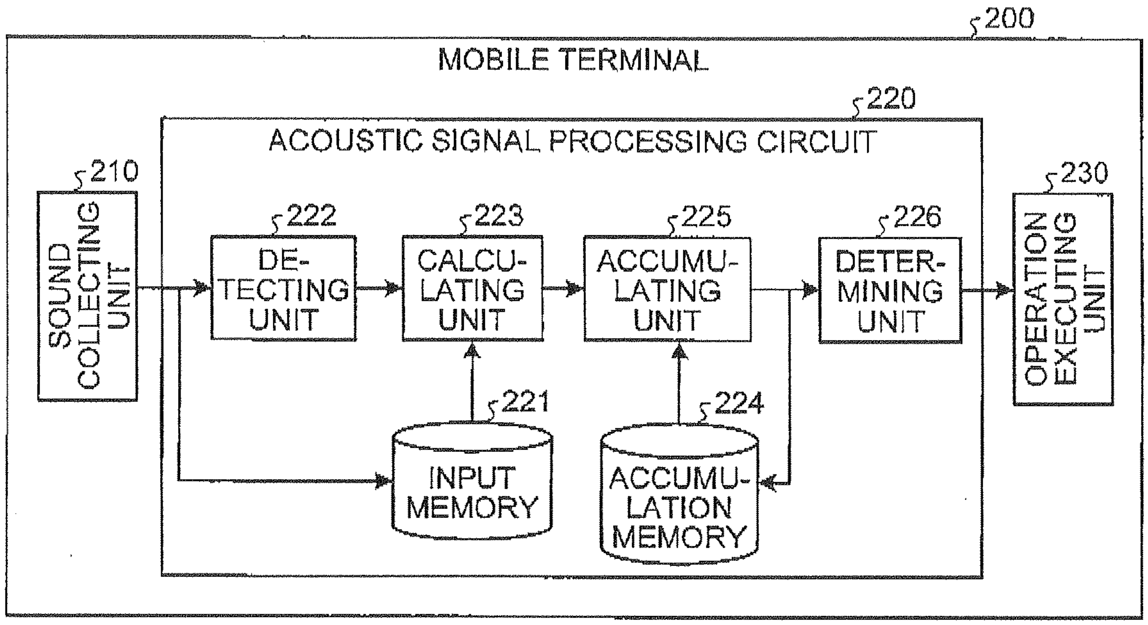
200
MOBILE TERMINAL
220
ACOUSTIC SIGNAL PROCESSING CIRCUIT
210
SOUND COLLECTING UNIT
222
DE-TECTING UNIT
223
CALCU-LATING UNIT
225
ACCUMU-LATING UNIT
226
DETER-MINING UNIT
230
OPERATION EXECUTING UNIT
221
INPUT MEMORY
224
ACCUMU-LATION MEMORY

FIG.16

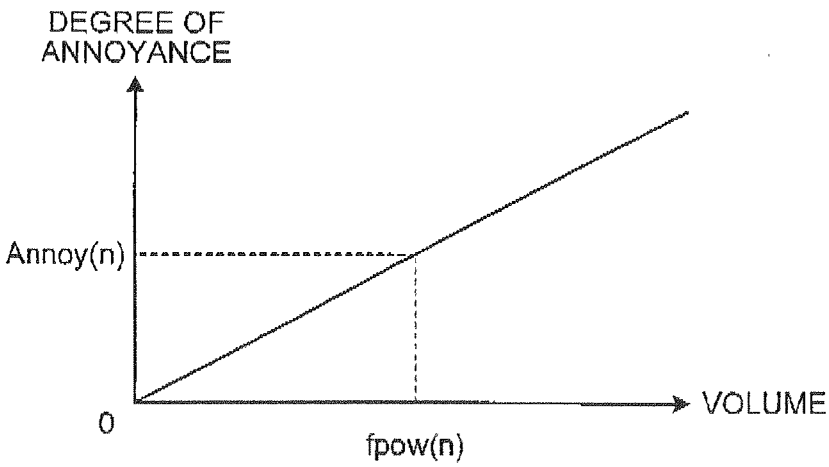
DEGREE OF ANNOYANCE
Annoy(n)
0
fpow(n)
VOLUME

FIG.17

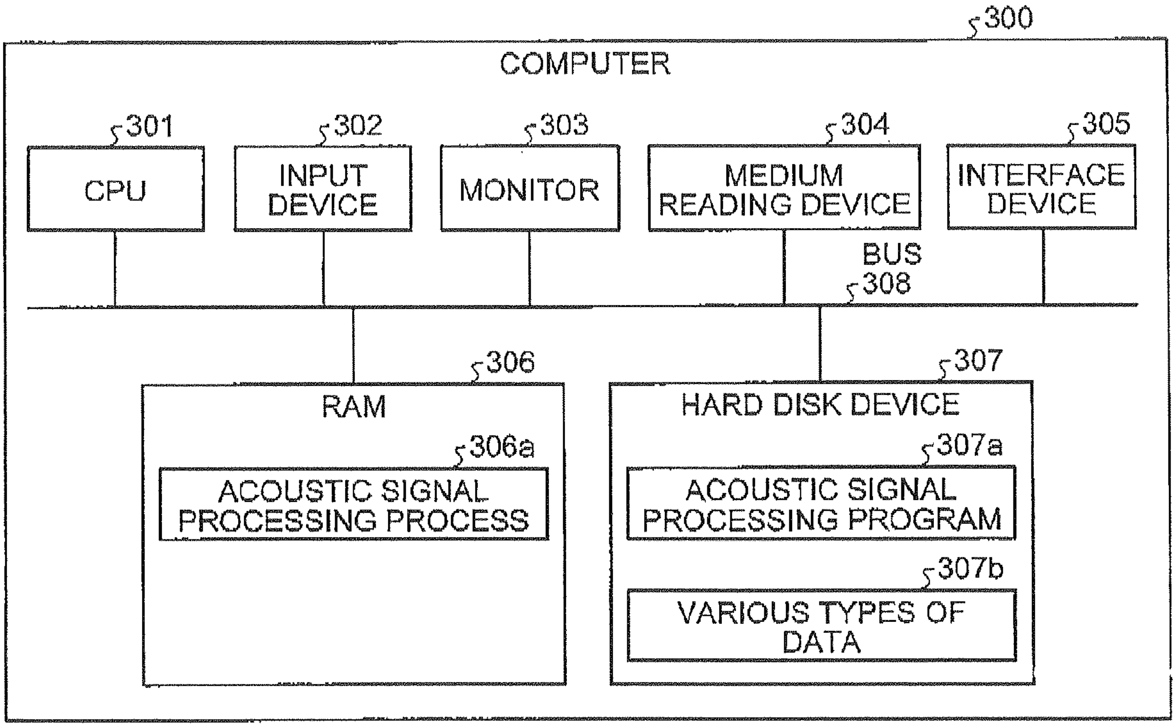
300
COMPUTER
301
CPU
302
INPUT DEVICE
303
MONITOR
304
MEDIUM READING DEVICE
305
INTERFACE DEVICE
BUS
308
306
RAM
306a
ACOUSTIC SIGNAL PROCESSING PROCESS
307
HARD DISK DEVICE
307a
ACOUSTIC SIGNAL PROCESSING PROGRAM
307b
VARIOUS TYPES OF DATA

FIG.18

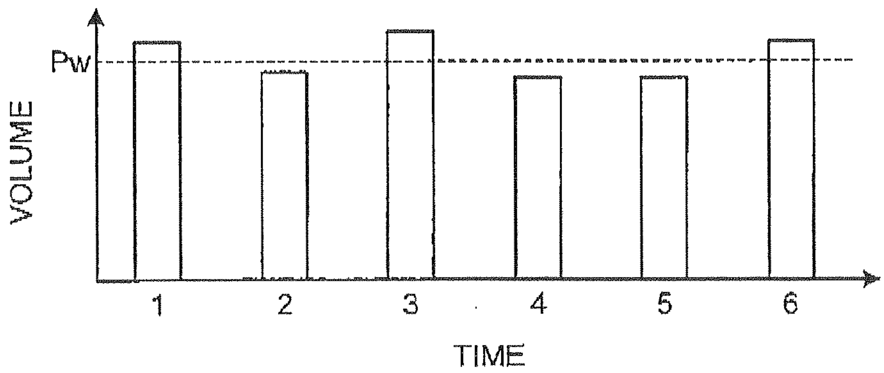
Pw
VOLUME
1
2
3
4
5
6
TIME

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3036078 B
- CA 1220283 A1
- US 2010283618 A1
- JP 7184948 A
- JP 2004187961 A